# EUROPEAN PATENT SPECIFICATION

(11) **EP 1 496 039 B1**
(45) Date of publication and mention of the grant of the patent: **14.02.2018**
(21) Application number: 03015879.4
(22) Date of filing: 11.07.2003
(51) Int. Cl.: C07B 61/00, G01N 33/53

(54) **A method for the generation of a dynamic combinatorial library**
Verfahren zur Erzeugung einer kombinatorischen dynamischen Verbindungsbibliothek
Méthode de création d'une chimiothèque combinatoire dynamique

(43) Date of publication of application: 12.01.2005
(73) Proprietor: Kirschfeld, Andreas, 69493 Hirschberg (DE)
(72) Inventor: Hochgürtel, Matthias, 69198 Schriesheim (DE); Eliseev, Alexey, Brookline MA02446 (US); Kirschfeld, Andreas Dr., 69493 Hirschberg (DE)

(56) References cited:
- EP-A- 1 184 359
- WO-A-03/033437
- NGUYEN RÉGIS ET AL: "Optimizing the reversibility of hydrazone formation for dynamic combinatorial chemistry." CHEMICAL COMMUNICATIONS (CAMBRIDGE, ENGLAND) ENGLAND 21 APR 2003, no. 8, 21 April 2003 (2003-04-21), pages 942-943, XP002265250 ISSN: 1359-7345
- HUC I AND LEHN J-M: "Virtual combinatorial libraries: dynamic generation of molecular and supramolecular diversity by self-assembly" PROCEEDINGS OF THE NATIONAL ACADEMY OF SCIENCES OF THE UNITED STATES, NATIONAL ACADEMY OF SCIENCE, WASHINGTON, DC, US, vol. 94, 1997, pages 2106-2110, XP002142245 ISSN: 0027-8424
- BUNYAPAIBOONSRI T. ET AL.: "Dynamic deconvolution of a pre-equilibrated dynamic combinatorial library of Acetylcholinesterase inhibitors" CHEMBIOCHEM, vol. 2, 2001, pages 438-444, XP002264584
- COUSINS ET AL: "Dynamic combinatorial libraries of pseudo-peptide hydrazone macrocycles" CHEMICAL COMMUNICATIONS - CHEMCOM, ROYAL SOCIETY OF CHEMISTRY, GB, no. 16, 1999, pages 1575-1576, XP002156551 ISSN: 1359-7345
- DATABASE WPI Week 200648, Derwent Publications Ltd., London, GB; AN 2006-469083 & KR 2005 0 052 929 A (POSTECH FOUND) 07 June 2005

## Description

The present invention relates to dynamic combinatorial chemistry (DCC), more precisely to a method for the generation of a dynamic combinatorial library (DCL) wherein one set of compounds forming the building blocks for the library are used in excess relative to the other compounds. The method is in particular appropriate for the reaction between compounds having an aldehyde functional group -C(O)H and compounds having a hydrazine functional group D-NRNH₂ carrying an electron withdrawing group D in α-position in the hydrazine group.

Biologically active compounds and medical drugs were traditionally generated by conventional chemical or biological methods by variation of so-called lead compounds. Since the generation of these compound variants is time-consuming, only a relatively small pool (a small library) of these compound variants is accessible for biological tests.

These problems can be overcome by combinatorial chemistry (CC), a method where a set consisting of various basis components is used to generate a variety of new compounds by successive and repetitive application of specific chemical reactions. The covalent nonreversible connections between the components are performed individually in parallel or concertedly in the same compartment(s). So, vast combinatorial libraries (CLs) of extensive collections of constituents are available in a very short time. CC is therefore a very powerful method for exploring the molecular geometrical and interactional spaces through molecular diversity generation and is thus based on large populations of different molecules that are present as discrete entities.

In contrast to this, the so-called dynamic combinatorial chemistry (DCC) is a conceptually different approach that requires a reversible assembly process and its constituents may be either molecular or supramolecular.

DCC thus relies on reversible reactions or interactions between sets of basic components to give access to dynamic combinatorial libraries (DCLs) of potential entities, allowing the target-driven generation or enrichment of the active compounds of the libraries. It thus represents a self-screening process by which the desired optimal species are preferentially expressed and retrieved from the DCL. DCC extends beyond static combinatorial chemistry (SCC) towards adaptive/evolutive chemical systems. It is especially useful for the search of ligands which bind to targets like enzymes, receptors or antibodies. Suitable detected ligands may be used as medicaments.

Several functional groups lend themselves for use in the generation of a DCL. Appropriate groups include aldehyde, keto, thiol, amino and hydrazine groups. The resulting molecules - which have been formed from at least two molecules present in the library - are linked by a unit formed in the reaction between the functional groups in the starting molecules. Examples are imines, hydrazones and disulfides.

DCLs can be generated in aqueous media as well as in organic solvents. They can be used for the generation and identification of ligands (in the presence of a given receptor) as well as for receptors (in the presence of a given ligand). For the identification of pharmacologically active compounds (which have an activity against a receptor, e.g. an enzyme), the DCL has to be generated and screened in an aqueous medium, with the pH preferably being close to neutrality, to mimic physiological conditions. Under non-physiological conditions or even in non-aqueous, organic media, the identification of active compounds may be hampered, due to the lack of stability of the target, or the compounds identified are not active *in vivo.*

When designing a DCL, two different procedures were employed hitherto. In one procedure a), library generating and screening is performed in one single step; in the other procedure b), these two steps are carried out separately. The particulars for the above-mentioned different procedures are the following:
a) adaptive, combinatorial libraries (one-step procedure): the generation of the library constituents is conducted in a single step in the presence of the target, so that the library composition may adjust leading to selection and amplification of the preferred substrate(s); screening by the target occurs in parallel with the reversible generation of the library constituents; this is the approach where the dynamic features are operative over the whole process;
b) pre-equilibrated dynamic combinatorial libraries (pDCL) (two-step procedure): the constituents of the library are generated by reversible interconversion and equilibration in absence of the target, which is added in a second step after reversibility has been stopped; this has the advantage that one may use reversible reactions which are not compatible with the presence of the target but the process is not adaptive and no amplification of the preferred substrate can result; it is however sufficient for lead generation, i.e. the discovery of species presenting the activity sought for; in its second phase it amounts to the usual, static combinatorial chemistry approach where an actual, real library is screened by the target; the resulting library may be termed pre-equilibrated or postdynamic combinatorial library (pDCL).

In practice, it has been shown that in many cases the generation of a DCL must be carried out according to procedure b), due to the following reasons. The reactions leading to the formation of a DCL, i.e. the initial formation of bonds by reaction between functional groups on the building blocks (from which the library is generated) and the reaction of the compounds thus generated with further building blocks or with other compounds thus generated, until equilibrium is reached, often is too slow under conditions where the target is stable. The library is thus generated under non-physiological conditions (e.g. low pH) until equilibrium is reached, within an acceptable period of time. The reaction conditions are changed thereafter such that the target is stable. The target is then added, and the library is screened.

In Proc. Natl. Acad. Sci. USA 1997, 94, 2106 - 2110 I. Huc and J.-M. Lehn disclose a method for the generation of a dynamic combinatorial library of imines from one set of aldehydes and one set of amines. The method is directed toward the synthesis of inhibitiors of the enzyme carbonic anhydrase by recognition-involved assembly. The synthesis of the above mentioned imines is carried out either in the presence of said enzyme (so-called adaptive combinatorial libraries) or said enzyme is added after equilibration (so-called pre-equilibrated dynamic or post-dynamic combinatorial libraries, pDCLs).

Quite similarly, in PCT application WO 03/033437 the generation of a dynamic combinatorial library is utilized to discover inhibitors of the enzyme neuraminidase. The method described therein makes use of amine-containing scaffolds which are e.g. reacted with an excessive amount of ketones at almost pH-neutral conditions whereby prior to its analysis the reaction mixture containing interchanging imines is continuously being quenched with a reducing agent. A consistent correlation between amplification factors and inhibitory activities could not be achieved, however.

The PCT-application WO 01/64605 discloses the *in situ* generation and screening of a dynamic combinatorial carbohydrate library against Concanavalin A. The plant lectin Concanavalin A belongs to the broad class of carbohydrate binding proteins. It is either present during library generation or is added after equilibration.

Hydrazines play an important role in the generation of DCLs, as they easily react with aldehydes and also ketones to hydrazones in a reversible reaction. This reaction is for example described in the above-mentioned publication by Huc and Lehn in Proc. Natl. Acad. Sci. USA 94, 2106 (1997).

The use of hydrazides NH₂NHC(O)- in DCC has been reported, see Sanders et al., Chem. Commun., 1761 (2000) and Chem. Commun. 1575, (1999). By the reaction with aldehydes, libraries containing macrocycles having acylhydrazone bridges are generated. The amplification (i.e. generation in higher amounts) of one or more components in the library can be attained by e.g. the addition of alkali metal ions. By the method according to Sanders et al., receptor molecules could be formed, for example for Li⁺-ions. Furthermore, the libraries are always generated in non-aqueous media, for example CHCl₃/MeOH.

Lehn et al. disclose (ChemBioChem. 2001, 438, and PCT-application WO 02/20435) a method for the generation of a DCL composed of interconverting acyl hydrazones which is screened towards inhibition of acetylcholinesterase. From a set of hydrazide and aldehyde building blocks a library was obtained. Of all possible acyl hydrazones formed, active compounds containing two terminal cationic recognition groups separated by an appropriate distance could be identified. The library was formed at a pH of 4.0, at which its formation occurs rapidly. The test of inhibitory activity was conducted at pH 7.2, at which the interconversion of the library constituents is essentially blocked.

However, the acylhydrazone formation required low pH-values of about 4, in order to be fully interconverting within a time period which is acceptable. Many targets, which in general are proteins, are however not stable under such non-physiological conditions. Therefore, the approach taken by Lehn was to firstly generate a dynamic library in the absence of the target, at a low pH-value, and then adding the target to the pre-equilibrated library at conditions close to pH 7. The dynamic library is thus not fully operative throughout the entire process of its formation, resembling the classical combinatorial chemistry approach.

It would be advantageous to have a method for the generation and screening of a DCL which does not have any static elements. This appears in particular important for the generation of DCLs in which scaffolds are used, as these in general contain several functional groups which should all be susceptible to reversibly interconvert with complementary functional groups on starting compounds present in the library.

The object of the present invention is to find a method for the generation and the screening of a dynamic combinatorial library DCL in an aqueous medium which method does not show the drawbacks of the methods as published to date. In particular, the library should be fully dynamic throughout the entire process comprising the generation and the screening of the library. The process should not require a pre-equilibration step. The static screening step, i.e. adding the target under non-reversible conditions, should not be necessary. It should be possible to carry out the process under physiological conditions (in aqueous media and a pH around 7). The process should lend itself for the generation of a library in which scaffolds are used.

These objects are attained by a method of forming a dynamic library of compounds which are potentially capable of binding to a target, which method comprises
i) selecting one first set of molecules which molecules each carry at least one first functional group which is capable of interacting with at least one other functional group under the formation of a reversible bond;
ii) selecting at least one further set of molecules which molecules carry at least one second functional group which is capable of interacting with the first functional group on the first set of molecules;
iii) reacting the two sets of molecules with each other and in the presence of the target, under conditions where a formation of reversible bonds between the functional groups on the molecules forming each of the sets occurs;
wherein a library containing products, that can be isolated and do not decompose when the set of molecules present in excess is removed, is generated,
the formation of the library is carried out in an aqueous medium at a pH of from 5 to 9 and is fully reversible throughout the entire formation of the library,
the formation of reversible bonds is the formation of hydrazones R¹C(R²)=N-N(R³)-D (I) by the reaction between a carbonyl function R¹C(O)R² (II) and a hydrazine function according to the general formula H₂N-N(R³)-D (III) wherein R¹ and R² are independently of each other hydrogen or an organic radical, R³ is hydrogen, an organic radical or an electron withdrawing group, and D is selected from the group -C(O)-, -C(O)-O-, -C(O)-S-, -C(O)-N<, -C(O)-C(O)-, -C(O)-(O)-O-, - C(O)-C(O)-N<, -C(S)-, -C(S)-S-, -C(S)-N<, -C(=N)-N<, -C(=N-CN)-N<, -C(=N-NO₂)-N<, -S(O)-, -S(O)₂-, -S(O)₂-O-, -S(O)₂-N<, -P(O)(-O-)(-O-), -P(O)(-O-)-, -P(O)-, or D forms together with the group R³ and the nitrogen atom to which it is attached a heterocycle,
and the respective amounts of each set of molecules employed are such that one functional group of that at least one second functional groups is present in an at least 5-fold excess with respect to the other functional group during the formation of the library, which means that an excess of hydrazine groups is used relative to carbonyl groups.

The method according to the present invention is in particular appropriate when a chemical reaction is employed for the formation of the library by which the exchange reaction between an existing chemical bond and a functional group present in the library occurs by a substitution mechanism.

In the context of the present invention, the term "large excess" means an at least 5-fold, preferably at least 20-fold, in particular at least 50-fold.

The set of molecules which is present in low amounts (low concentration) determines the maximum concentration of compounds to be produced in the DCC reaction while the set of molecules at higher concentration ensures a minimum rate of equilibration. The first set of molecules is employed in a concentration which is close to the concentration of the target, in general in the low µM range, preferably about 1 to 100 µM. The other set of molecules is used in excess to maintain equilibrium rates. The concentration of the second set is usually in the higher µM to the low mM range, preferably about 500 µM to 10mM.

The method according to the present invention allows to generate libraries containing stable products. In the context of the present invention, "stable" means that the products can be isolated and do not decompose when the set of molecules present in excess is removed. In contrast, the formation of imines by the reaction between aldehydes and amines gives products which are only stable in the presence of a large excess of one set of starting products. In order to analyze the library (detect the products), the products formed have to be converted into stable species. In the case of imines, this is achieved by hydrogenating these into amines. The formation of DCC's by reactions which do not result in stable products in the sense of the definition given beforehand is not a part of the present invention.

Miller et al. (Tetrahedron Letters 38, 1997, pp 8639 to 8642 describe the formation of a dynamic combinatorial library wherein various salicylaldimines are reacted with a 10- to 50-fold excess of Zn²⁺.

Caran and Miller (Journal of the American Chemical Society 123, 2001,pp 7455 to 7456) disclose the formation of a dynamic combinatorial library wherein the binding of salicylaldimines to an RNA sequence in the presence of Cu²⁺ is used.

A molecule belonging to one set of molecules used for the generation of the library carries at least one "functional group" which term designates a group capable of reacting with another functional group, which may be identical with or different from the first functional groups, under the formation of a reversible chemical bond. The molecules carrying a functional group thus are the "building blocks" from which the (active) components are formed.

Examples for functional groups which may react with other functional groups under reversible covalent bond formation include amino groups, aldehyde groups, keto groups, thiol groups, olefinic groups, alcohol groups, ester groups, carbonyl groups, hydrazine groups, hydroxylamine groups and borate groups. Examples of reversible covalent reactions with the above-mentioned groups involved are those where carbonyl groups react under the formation of imines, acylhydrazones, amides, acetals, and esters. In particular, the reaction of amino groups with carbonyl groups to imines, oximes or hydrazones is useful. Reactions such as thiol exchange in disulphides or alcohol exchange in borate esters or carboxylate esters are further examples for preferred embodiments, as well as reversible Diels-Alder and Michael reactions or alkene metathesis. Photoinduced interconversions represent another possibility leading to photodynamic combinatorial processes.

The excess values given above always refer to the entire set of functional groups. When, for purposes of illustration, the molecules in the first set of molecules carry one first functional group and the molecules in the second set of molecules carry two of the second functional group, and the first functional group would be used in excess, the first set of molecules would have to be used in a 10-fold excess in order to reach a 5-fold excess of the first functional group relative to the second functional group.

The first functional group and the second functional group are as defined above. As mentioned beforehand, the method according to the present invention lends itself in particular for the generation of a DCL by the reaction between functional groups initially resulting in the reversible formation of a chemical bond and wherein the exchange or reverse reaction (i.e. the reaction with a functional group under the formation of the same type of bond involving a new molecule and releasing one of the reaction partners previously bound) is a substitution reaction. That means that the chemical bond initially formed will not be broken and then a new bond be formed by the reaction between a molecule previously not involved and one of the molecules involved in the formation of the previously formed initial chemical bond; the new chemical bond will instead be formed by the attack of a molecule previously not involved in the formation of the initial chemical bond, formation of a new chemical bond and subsequent release of one of the molecules involved in the formation of the initially formed chemical bond.

The functional group which is used in excess preferably is the functional group which attacks the chemical bond formed in the substitution reaction, under the formation of a new chemical bond. Examples will be given below.

When in the context of the present invention reference is made to a "first" functional group, a "second" functional group and so forth (meaning that there can be a "third", "fourth", "fifth", ..... functional group), this means that the "first" functional group is a defined functional group (e.g. a carbonyl group) present in every molecule of the first set of molecules. This likewise applies to the "second" functional group and so forth.

In one embodiment of the present invention, the molecules for which the first, second, .... set of molecules is formed contain only one defined functional group.

The molecules may also contain, in a further embodiment, more than one (i.e. two, three, four, five, ...) functional groups. In the latter case, the functional groups present on a molecule may be identical functional groups (e.g. "a first functional group"), or the functional groups may be selected from a group of two, three, four, ... different functional groups. The latter is in particular the case when a scaffold is used in the generation of the library. In this case, one set of molecules employed in the formation of the library is formed by the scaffold.

It is preferred when the scaffold carries at least one aldehyde group and the other set of molecules carries a hydrazine group and is used in excess.

When in the context of the present invention the "generation" and the "screening" of a DCL are mentioned, one should be aware that these two steps can often not be separated in the formation of a DCL. For example, it is possible to mix the starting compounds with each other in the presence of the target which is then active in the entire process, meaning that the generation and the screening occur simultaneously. It is also possible, for example, to generate the library in the absence of the target, until equilibrium is reached, and then add the target. Although it can be said that the first step is the generation of the library and the second step the screening of it, this is not unambiguous as in the presence of the target the library is reformed or reequilibrated (i.e. the relative proportions of the components change, due to reversibility of the library-forming process), meaning that the generation of the DCL here also occurs, simultaneously.

The term "formation" of the library, as used in the context of the present invention, includes both steps, i.e. the generation and the screening of the library.

It was found that a DCL can be formed in an aqueous medium, in particular an aqueous medium, which is close to or even at pH 7 and, hence, under physiological or almost physiological conditions, in the presence of the target, when the above-mentioned parameters are respected. The target - which in general is a biological target from the group of proteins - is stable under these conditions.

In the context of the present invention "close to pH 7" means a pH of from about 5 to 9. The value of choice depends on the stability of the specific target which may show its maximum activity at a pH of about 5.5 or of about 8. It is preferred to carry out the generation of the library at a pH of from about 5.5 to 8.

Preferably, the formation of the library is carried out in a buffered solution.

The dynamic process is fully operative throughout the entire process. The library can be formed first in the absence of the target, which is subsequently added to cause the enrichment of the active compounds. As already mentioned above, the first step could be regarded as the generation of the DCL, whereas the second step could be regarded as the screening thereof. The library can also be generated directly in the presence of the target.

When designing a dynamic library making use of the method according to the present invention, the following procedure can hence be employed. The generation of the library is conducted in a single step in the presence of the target, so that the library composition may adjust leading to selection and amplification of the preferred component(s); screening by the target occurs in parallel with the reversible generation of the library constituents; the molecules (building blocks) from which the library is formed and the resulting components can enter into contact with the target from the beginning:
In any case, the process according to the present invention allows the dynamic parameters to be fully operative throughout the entire formation of the library. Of course, the process may be carried out such the dynamic parameters are temporarily interrupted, as long as in the formation of the library an equilibrium can be reached. The dynamic process may also be interrupted after the library has attained equilibrium, for example in order to convert unstable into stable compounds before the analysis of the library.

In a preferred embodiment of the present invention, the molecules in at least one set of molecules used for the generation of the library carry a functionality. The term "functionality" used in the present invention designates a unit which can bind to the binding sites of the target, or interact with the target otherwise than by binding to it, for example by steric interactions.

"Functionality" means any polar, nonpolar, hydrophilic or lipophilic, or charged unit or subunit or electron donor or electron acceptor group. "Functionality" on the one hand includes simple functionalities like amino and imino groups and derivatives thereof, hydroxy and mercapto groups and derivatives thereof, oxo and thioxo groups, formyl and thioformyl groups, aryl groups, substituted aryl groups, phenyl groups, substituted phenyl groups, pyridyl groups and derivatives thereof, carboxy groups and carboxylato groups and derivatives therof, alkyloxycarbonyl groups, (di)thiocarboxy groups and derivatives thereof, (di)thiocarboxylato groups, carbamoyl groups and derivatives thereof, sulfo, sulfino and sulfeno groups and derivatives thereof, alkyloxysulfonyl, alkyloxysulfinyl and alkyloxysulfenyl groups, sulfamoyl, sulfinamoyl and sulfenamoyl groups and derivatives thereof, cyano and (iso)(thio)cyanato groups, hydroperoxy groups, nitroso groups, hydroxyamino groups, hydrazino groups, -NR¹R², -⁺NHR¹R² and -⁺NR¹R²R³ groups, wherein R¹, R², and R³ are identical or different and represent alkyl, cycloalkyl, alkylcycloalkyl, aryl, alkylaryl with 1 to 40 C atoms, -⁺OR¹R² groups wherein R¹ and R² are identical or different and represent alkyl, cycloalkyl, alkylcycloalkyl, aryl, alkylaryl with 1 to 40 C atoms, hydrazide groups and any other suitable groups known to a person skilled in the art.

On the other hand, "functionality" also includes more complex components, and non-limiting examples include heterocycles carrying one or more heteroatoms in the ring selected from the group consisting of N, O and S, amino acids and oligo- and polypeptides, sugars (preferably hexoses and pentoses), sugar derivatives (like peracetylated sugars) and oligomers and polymers thereof, and nucleic acids and derivatives thereof.

The functionalities can thus be linked to a molecule, i.e. a molecule carrying the functionality (functionalities) is formed; the functionality can also itself be the molecule (and thus form the molecule carrying the functionality). An example of the latter case is a sugar molecule.

When the molecules carrying a functional group combine with each other, "components" are formed. These components preferably already present the "ligands" which may have functionalities and which bind to the target. As according to the present invention the library should be fully reversible throughout the entire process of its formation (in the absence and in the presence of the target), no reaction should be necessary to transform the components to the ligands like, for example, the reduction of generated imines to amines.

The ligands (i.e. the components which bind to the target) are also referred to, in the context of the present invention, as "pharmaceutically active compounds".

"Component" (ligand) means a molecule with a molecular weight typically not greater than 1500, preferably not greater than 1000, advantageously not greater than 500, which possesses an affinity for a target, i.e., that is able to interact with the target by forming one or a plurality of weak bonds such as hydrogen bonds, hydrophobic interactions, charge-charge interactions, Van der Waals interactions, donor-acceptor interactions, charge-transfer interactions, metal ion bindings, etc.

"Target" means a biological or synthetical macromolecule with a molecular weight typically greater than 5000. Biological macromolecules may be proteins including lipoproteins, glycoproteins and analogues of proteins, wherein either the peptide bond -CO-NH- is replaced by an analogous bond, possibly reversible such as an imine, ester, sulfonamide, sulfone, sulfoxide, phosphate, phosphonate, phosphonamide, guanidine, urea, thiourea, or imide bond, or wherein the aminoacids are replaced by synthetical derivatives thereof. The natural proteins may have differing functions, they may act namely as enzymes, as receptors or as antibodies. Receptors may be membrane receptors, hormone receptors, or signal transducers.

If the target is an enzyme, the ligand which is sought to be obtained may act as a substrate, an inhibitor or an activator for said enzyme.

If the target is a receptor, the ligand which is sought to be obtained may act as a natural or artificial ligand, an agonist or an antagonist for said receptor.

If the target is an antibody, the ligand which is sought to be obtained may act as an antigen for said antibody.

In general, all kinds of molecules, the one of which can act as a ligand and the other one as a target, are suitable to be used in the method according to the present invention.

The scaffold can be any molecule which carries at least one functional group, preferably at least two functional groups, and which has a structure which can potentially form the basis of an active component for a given target. In order to transform the scaffold into a component, the functionalities have to be attached to the scaffold. In a DCC-screening process, this occurs via the formation of reversible bonds by the interaction of functional groups on the molecule carrying the functional group and the scaffold. The scaffold can itself already have a certain activity with respect to the target, in general a biological activity. The scaffold can also show no activity. However, in any case, it is only after the attachment of functionalities to the scaffold that a lead compound, having a desired activity, is generated.

In one embodiment of the present invention, the scaffold is selected according to the requirements given by the binding site of the target. These requirements can be with respect to a certain size, three-dimensional structure and/or flexibility of the scaffold. It is mandatory that the scaffold can be easily controlled and operated under mild conditions. Furthermore, the compatibility with the bonding of the functionality to the target is an important feature of an appropriate scaffold for a given target. By the right choice of a scaffold having an appropriate size, three-dimensional structure and/or flexibility, the compounds formed, respectively the functionalities on the compounds, will fit into the binding site of the target and interact with it. One method to establish the appropriate size of the scaffold is, for example, analyzing the size of the binding site by methods known in the art like, for example, by X-ray structural analysis. It is also possible, for example, to evaluate the size of a known ligand for a given binding target and mimic this size.

The scaffold is a small organic or inorganic molecule. Organic molecules are preferred. In the context of the present invention, the term "small molecule" denotes a molecule which has a molecular weight in a region typical for organic molecules having a pharmacological activity and which are synthesized by chemical methods or are accessible by chemical methods. Preferably, the small molecules have a molecular weight of below about 500. The term "small molecule" does not refer to molecules of a high molecular with a pharmacological activity and which are typically synthesized by biochemical methods.

"Organic molecules" are molecules which contain at least one unit in the structure which is derived from the units -CH₃, -CH₂- or ≡CH wherein the hydrogen can partially or totally be replaced by substituents which can form a bond to the remaining carbon atom. Such substituents are known to the person skilled in the art and include, for example, halogen atoms, amino groups, ogygen atoms, hydroxy, alkoxy and aryloxy groups, thiol groups, organophosphine groups and organosilicon groups.

The organic molecule representing the scaffold can be of a cyclic or non-cyclic structure and can contain one or more heteroatoms, preferably from the group consisting of N, O, S, P, B, Si and halogens. The scaffold can also contain one or more double or triple bonds. The scaffold contains in its hydrocarbon chain at least one carbon atom, preferably at least three carbon atoms, in particular four to ten carbon atoms. The scaffold furthermore carries at least one functional group, preferably two to six functional groups, more preferably two to four functional groups, in particular three or four functional groups which are selected from the group cited above in connection with the molecules carrying functional groups. The functional groups do not belong to the scaffold, as the term is understood in the context of the present invention. The scaffold can have one or more functionalities which are in accordance with the definition given above.

Scaffolds and their use in DCC are described in the application WO 03/034071.

Preferably, the reactions used in the present invention allow to lock the process by a simple reaction or by changing conditions of the equilibrium.

In all embodiments of the formation of the DCL according to the present invention, the target can be used in a soluble form. The target can also be present in immobilized form, for example bound to a solid support. After the generation of the library and the addition of the target (which is present either during the generation of the library or added afterwards, as laid out above) the reaction mixture containing the library is submitted to a work-up procedure which can be carried out in the following ways:
A) The target with the components bound to it is separated from the reaction mixture by methods known to the person skilled in the art. In the case of immobilized targets, the target is simply taken out of the mixture or filtered off. In the case of a soluble target, the target-component adduct can e.g. be removed by filtration techniques, in particular by ultrafiltration. After the separation of the target-component adduct the components are released. This can occur by destroying the target, for example by denaturation, in the case of a protein. It is sometimes also possible to release the component by changing the pH of the mixture. In particular, in the case where the target is a protein and protein-protein interactions are inhibited, a pH-change, for example by re-dissolving the target in a buffer solution, will release the bound components. After the release, the components are identified.
B) The target remains in the reaction mixture. It may not be necessary to release the bound components from the target, for example in cases when the target can promote formation of preferred species with a turnover. The release of the components is however necessary in many cases. This is generally done with the methods known to the person skilled in the art, in particular with the methods which were described under A). In cases where the components are released, such release can occur simultaneously with the conversion of the transient species into stable species.

In order to identify the compounds which preferably bind to the target and which are hence preferably formed in the presence of the target, several possibilities exist. In one embodiment, the library formed in the presence of the target is analysed (i.e. the compounds formed are identified by analytical methods known to the person skilled in the art, preferably by mass spectrometry) and compared with a library formed under identical conditions, but in the absence of the target. In the context of the present invention, the library obtained after the "formation equilibrium" will thus in general be compared to the library obtained after the "exchange equilibrium". The compounds which preferably bind to the target are those which are formed in higher amounts in the presence of the target. This embodiment is preferred when the target is used in soluble form. The target is generally destroyed when the mixture is analysed, in order to release the target-bound fraction.

It is also possible to isolate the target-ligand complex from the mixture, release the active compound (ligand) from the target and analyse the ligand. This is preferred in cases the target is used in immobilised form.

The present invention lends itself in particular for the generation of a DCL using the formation of hydrazones R¹C(R²)=N-N(R³)-D (I), wherein D is an electron withdrawing group, by the reaction between a carbonyl group R¹C(O)R² (II) and a hydrazine H₂N-N(R³)-D (III), wherein D is an electron withdrawing group.

As the present invention relates to a general process for the generation of a DCL, it is not appropriate and even impossible to define the organic residue carrying the functional group(s) of the formulae (I), (II) and (III) exactly, as all kinds of molecules carrying various functional groups and/or heteroatoms belonging to the various classes of organic molecules which are known to the person skilled in the art can be used in the generation of a DCL. This is even more evident when it is considered that the organic rest can be a scaffold to which the functional group(s) is or are attached, which is one embodiment of the present invention. R¹, R² and R³ are thus not restricted and can be hydrogen or any appropriate organic rest.

Examples for organic rests include saturated and unsaturated, linear and branched, cyclic and acyclic, substituted and unsubstituted aliphatic groups, which groups may contain hetero atoms, as well as mono- and polynuclear, substituted and unsubstituted, optionally hetero atom containing aromatic groups. Suitable aliphatic groups may contain 1 to 100 carbon atoms, preferably 1 to 50 carbon atoms, in particular 1 to 20 carbon atoms. Suitable aromatic groups may contain 3 to 30, preferably 4 to 20 carbon atoms. R¹ and R² can also form together with the C-atom to which they are attached a ring which may be substituted or unsubstituted and may carry one or more hetero atoms in the cycle.

Appropriate electron withdrawing groups D are known to the person skilled in the art. Non-limiting examples include -C(O)-, -C(O)- O-, -C(O)-S-, -C(O)-N<, -C(O)-C(O)-, -C(O)-(O)-O-, -C(O)-C(O)-N<, -C(S)-, -C(S)-S-, -C(S)-N<, -C(=N)-N<, -C(=N-CN)-N<, -C(=N-NO₂)-N<, -S(O )-, -S(O)₂-, -S(O)₂-O-, -S(O)₂-N<, -P(O)(-O-)(-O-), -P(O)(-O-)-, -P(O)-.

-C(O)- is preferred.

In one embodiment of the present invention, R³ is a further electron withdrawing group which can be identical with D or different therefrom.

In a further embodiment of the present invention, D forms together with group R³ and the nitrogen atom to which it is attached a heterocycle. The heterocycle may contain one or more further hetero atoms and has electron-delocating properties. The heterocycle is an aromat, for example.

Suitable substituents are aliphatic groups, aromatic groups, halides, oxygen, sulfur, amino and organyl amino groups, nitro groups, carboxyl groups, ester groups, alcohol groups, thiol groups.

Hetero atoms include B, N, Si, P, O, S.

Nguyen and Huc disclose in Chem. Commun. 2003, p. 942 to 943 the generation of a DCL by reacting hydrazones carrying electron withdrawing goups, inter alia hydrazides, and aldehydes to hydrazones or acyl hydrazones, respectively. The library is generated at pH-values of from 6 to 8 and appears to be fully reversible. One experiment is described wherein an excess of CF₃-C(O)-NH-NH₂ (20-fold) is employed in the formation of a DCL; the aldehyde employed was isobutyraldehyde. This reaction is not a part of the present invention and is not used for the generation of a DCL according to the present invention. Huc et al however do not describe or give any hint that the excess of the hydrazide permits the library to be fully reversible throughout the entire formation process. This observation is attributed to the electron withdrawing group which is present in the hydrazones and hydrazides used (like a -C(O)-CF₃ group in the example given above).

The dynamic exchange (chemical reaction) between a hydrazone group already formed and a hydrazine function can be achieved, according to the present invention, by using an excess of hydrazine with respect to the hydrazones. As the hydrazone is formed from carbonyl and hydrazine groups, this means that an excess of hydrazine groups is used relative to carbonyl groups.

In a preferred embodiment of the present invention, aldehyde is used as a carbonyl function.

The excess of hydrazine groups necessarily depends on the individual compounds of which the library is formed. The relative amounts can be determined by standard methods by the person skilled in the art. In general, the hydrazide groups have to be present in a 5-fold, preferably 10-fold, and particularly 50-fold excess, relative to the aldehyde groups present.

As the present invention applies to monofunctional as well as to bi- and polyfunctional molecules (e.g. scaffolds), the excess is always calculated relative to the amount of a given functional group, not to the molecules as such, as has been laid out above.

In the context of the present invention, the target can be a ligand or a receptor. Preferably the target is a receptor, with the molecules formed being ligands for the receptor.

Examples for appropriate hydrazines include those according to the following formulae:

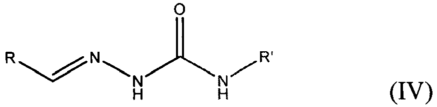

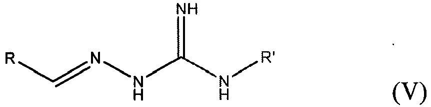

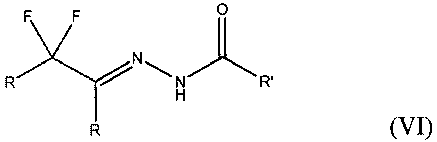

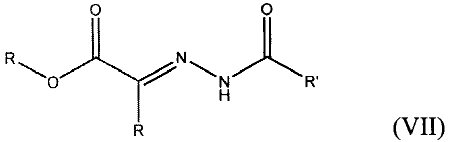

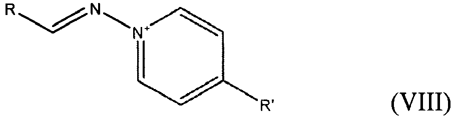

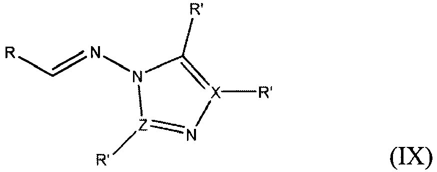

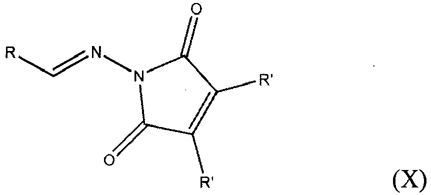

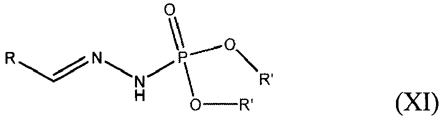

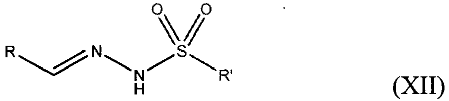

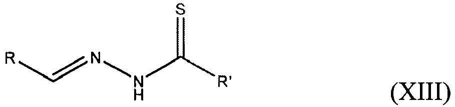

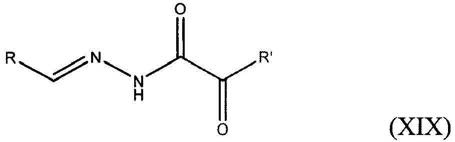

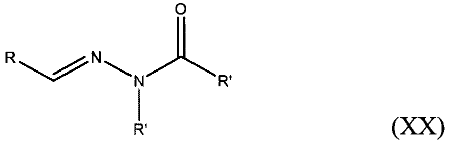

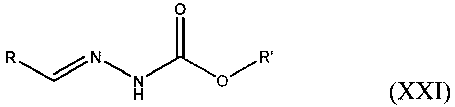

In the formulae (IV) to (XXI), the groups R and R' can be any appropriate organic group known to the person skilled in the art. R and R' can have the same meanings as mentioned by way of example in respect to groups R¹, R² and R³. Furthermore, in the formula (IX), X and Z can be =CH- or -N=.

In one embodiment of the present invention, unmodified aldehyde groups are employed. In the context of the present invention, the term "unmodified aldehyde groups" designates aldehyde groups in which the alkyl or aryl group in □-position to the carbonyl group do not carry a further functional group or a hetero atom. The chemical behaviour with respect to the reaction with a nucleophile should essentially be identical to that of an aliphatic or aromatic aldehyde carrying no further substituents.

The aldehyde group can however be chemically modified in a way which does not alter the chemical behaviour relative to the attack of a nucleophile. Examples for such modified aldehyde groups are acetal groups including hemiacetal groups.)

A further object of the present invention is a method of synthesizing and identifying a pharmaceutically active compound, which method comprises forming a library according to any of the methods of the invention as described above, analysing the library by methods known as such and identifying the compound or compounds which preferably bind to the target.

In one embodiment of this object, the compound(s) binding preferably to the target is/are identified by comparing the amount of one or more compounds formed in the absence of the target with the respective amount formed in the presence of the target.

A final object of the present invention is a method for the manufacture of a pharmaceutical preparation, comprising synthesizing and identifying a pharmaceutically active compound according to any of the methods of the invention as described above, optionally synthesizing and isolating the compound in amounts necessary for a medicament, and formulating the active compound and a pharmaceutically acceptable carrier into a pharmaceutical preparation.

Optionally, the pharmaceutically active compound has been synthesized and isolated by methods known as such in amounts sufficient for the manufacture of a medicament.

The invention is now illustrated in the following examples.

### Example 1

The target is a cell surface receptor protein (in its soluble form) which binds with high affinity to its natural ligand, an extracellular protease, via relatively large and discontinuous, multiple-domain molecular surface interactions. Disruption of this protein interaction was put forward as a new therapeutic concept for cancer treatment. Therefore the target has gained attention over the recent years. Aside from large cyclic peptides that were derived from the binding epitope of the natural ligand, 5 classes of small synthetic inhibitors were already known from the patent literature.

One of the known inhibitors consists of a mixed peptide-peptoid backbone with a m.w. < 600 which is more or less conserved among the majority of the published structures. This backbone is usually connected via an alkyl or alkylamino spacer to an additional residue of low m.w. (< 200) and larger variability.

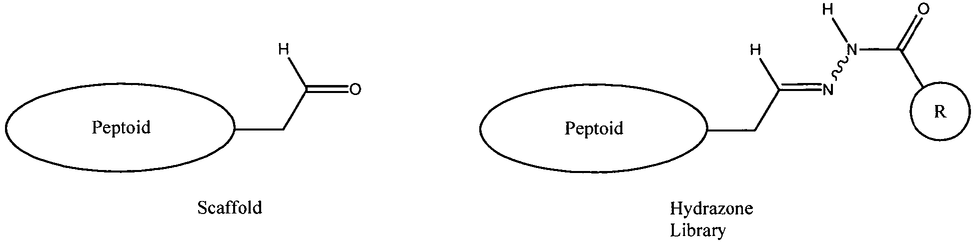

A scaffold was synthesized in which the peptoid backbone was modified with a reactive aldehyde group (schematically represented above). Since biological activities of such reactive compounds are not meaningful, a derivative in which the aldehyde C=O bond had been replaced by a C=C bond was tested. Although it was known that the backbone should contribute largely to the overall affinity of the final products, this scaffold mimetic, which lacks a significant functional R group, showed only weak and incomplete inhibition. By reaction with a set of small acyl hydrazides the scaffold was then converted into a library of hydrazones in which all components can convert into each other.

The method was validated and the experimental conditions optimized using a 2-component hydrazone mixture. The components **A** and **B** (hydrazones) were chosen according to SAR assumptions based on imine DCC chemistry with the same target and on literature data. **A** was derived from a hydrazide that contained a structural element similar to that of a known inhibitor while **B** contained a simple methyl substituent only which was expected not to be recognized particularly by the protein. The biological activity of **A** was later confirmed to be ca. 600 nM, that of **B** ca. 5 µM.

**A** could be identified in all DCC reactions as the more active component, even under unfavourable circumstances. For example, in order to rigorously establish a thermodynamic relationship as the underlying principle for selection, the hydrazide building blocks **A'** and **B'** corresponding to **A** and **B** were mixed in an **A':B'** ratio of 1:10. Nevertheless, while **A** proved to be only a minor part in the blank run it was selectively enriched in the presence of the target until it dominated the mixture (whereas **B** was deselected). Enrichments by a factor of ca. 3 were typical in such experiments which is far above the achievable analytical precision and reproducibility errors.

Enrichment factors were typically determined relative to parallel runs using a target which was selectively blocked by either a part of the natural ligand or a known synthetic inhibitor. Additional reference experiments were always run with blank (protein-free) samples as well as with samples containing an indifferent protein (typically BSA).

In order to determine the scope of the method several libraries containing ca. 10-12 compounds each were prepared. The components were chosen for either broad or focused diversity or for strong or weak binding, and combinations of both criteria. The largest library made until now contained 45 different hydrazones (5 parallel runs of 9 each) which were chosen from the group of phenylacetic acid and related derivatives. Each grouping of 9 was additionally spiked with hydrazone **A** as a common reference component. Only hydrazones that were selected to an extent higher than **A** were taken as hits. The nine best hits from all 5 sublibraries were later pooled together in a new library, among which only one showed up with outstanding enrichment.

### Example 2 Reversible exchange between nitrone and aldehydes

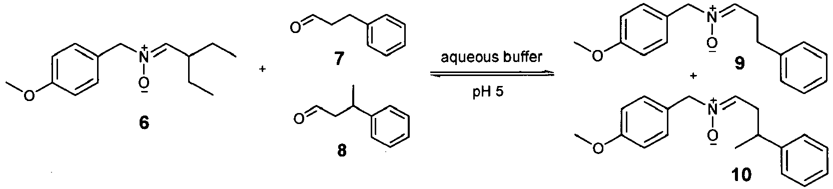

Nitrone **6** was synthesized following literature procedure (Dondoni, A. Synthesis of N-Benzyl Nitrones, Synthetic Communication, 1994, 24 (18)) by condensation of 2-ethyl-butyraldehyde (**3**) with *N*-(4-Methoxy-benzyl)-hydroxylamine. To a solution of crude aldehyde in chloroform was added the hydroxylamine followed by a large excess of magnesium sulphate. The reaction mixture was stirred at room temperature for 2.5 h and filtered. The filtrate was evaporated to yield a colourless solid. Identity and purity of crude nitrone was checked by ¹H-, ¹³C-NMR and HPLC/MS. ¹H-NMR (CDCl₃) □ 7.25 (d, 2H), 6.85 (d, 2H), 6.35 (d, 1H), 4.75 (s, 2H), 3.72 (s, 3H), 2.88 (m, 1H), 1.35 (m, 4H), 0.76 (t, 6H); ¹³C-NMR (CDCl₃) □ 160.4, 143.3, 131,1, 125.6, 114.6, 69.3, 55.7, 39.3, 24.8, 11.9; LRMS (ESI+) m/z 236 ([M+H]⁺). For DCC related experiments a 62 mM stock solution was prepared by dissolving 11 mg nitrone 6 in 740 µl DMSO.

The reversible exchange between synthesized nitrone and a mixture of aldehydes was tested by following experiment. To a 31 µM solution of nitrone **6** in ammonium acetate buffer (10 mM, pH 5, 2 ml) was added aldehyde **7** and **8** (1 µl of 1M solution in DMSO each). After incubation for 24 h the reaction mixture was analysed by HPLC/MS. MS data were acquired in full scan mode and scanned for protonated molecular ions [M+H]⁺ of potential nitrones in extracted ion chromatograms (EICs). A distribution of all possible nitrones could be detected. LRMS (ESI+) m/z 236 ([M+H]⁺); m/z 284 ([M+H]⁺); m/z 270 ([M+H]⁺).

### Example 3 Reversible exchange between nitrone and hydroxyl amines

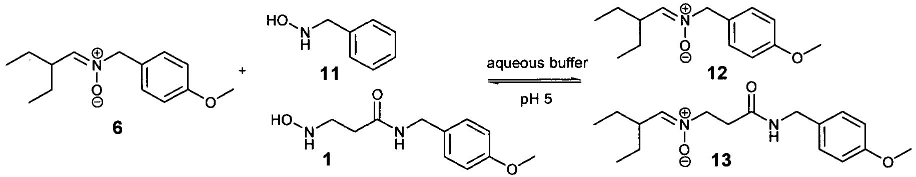

The reversible exchange between synthesized nitrone and a mixture of hydroxyl amines was tested by the following experiment. To a 31 µM solution of nitrone **6** in ammonium acetate buffer (10 mM, pH 5, 2 ml) was added hydroxyl amine **1** and **11** (4 µl of 62 mM solution in DMSO each). After incubation for 24 h the reaction mixture was analysed by HPLC/MS. MS data were acquired in full scan mode and scanned for all potential nitrones by generation of extracted ion chromatograms (EICs). A distribution of all possible nitrones could be detected. LRMS (ESI+) m/z 236 ([M+H]⁺); m/z 206 ([M+H]⁺); m/z 307 ([M+H]⁺).

Identity of new nitrone products was additional check by MS/MS experiments.

### Examples

### Materials and Methods

### Library Preparation

Mono- and polyvalent scaffolds used in the following experiments are by definition denoted as **SI, SII,** ... while monofunctional hydrazide molecules are being numbered as **H1, H2,** ... **(Hn).** The condensation products of scaffolds and molecules (hydrazones) are given the combined number of both building blocks from which they were formed as an identifier (e.g. **SI-H23).**

Equilibration in the presence of a target is usually carried out in 10 - 20 mM ammonium acetate buffer at pH 5.5 - 6.0 unless otherwise stated. Typical target concentrations used in DCC experiments are in the range of ca. 26 - 31 µM. The target: scaffold ratio is usually kept at a molar ratio of ca. 1.5 : 1 while ratios between 0.5 : 1 and 3 : 1 have also been tried successfully. All experiments shown here were carried out at room temperature although DCC experiments at lower (5 °C) or higher (40 °C) temperature gave similar results. Equilibration is complete within a few hours for libraries of small size, low affinity and high excess of hydrazides but can take a few days for large libraries of strong binders. A typical experiment with a 10-component library is finished within about 1 - 2 days.

The fully equilibrating DCC mixtures can either be analysed as such at certain time intervals by HPLC/MS or aliquots are taken and the equilibration is stopped in these aliquots before injection onto the column via addition of a small volume of 0.1 M aqueous ammonia which causes a pH shift to ca. 9 - 9.5. It is also possible to separate the protein-bound fraction of the DCC mixture prior to analysis by common rapid ultrafiltration or chromatographic methods which separate according to molecular size.

A DCC experiment is usually accompanied by at least 3 control runs in which the target solution is replaced by a) an equivalent volume of pure buffer, b) an equal volume of the target solution in which the binding site is specifically blocked by a high-affinity inhibitor, and c) an equal volume of a protein solution of equal concentration which is believed to behave essentially inert towards all library components or at least not to bind them in a specific manner (e.g. bovine serum albumin, BSA). The target-inhibitor complex under b) is preformed by incubation of the target solution and with at least one molar equivalent of the inhibitor (mM stock solution in DMSO) for about one hour prior to addition to the DCL. As an inhibitor either the natural ligand of the target, a part thereof or a synthetic antagonist with a known affinity for the target could be used. In the experiments presented here, only synthetic antagonists for which activity data had been published or had been verified internally were employed. Enrichment percentages given in the following examples are always determined relative to such control experiments. Unselectively enriched components can also be identified using this method.

### Library Analysis

Typical HPLC/MS method for large dynamic libraries with target, especially with target separation:
HPLC-MS analyses were performed with a Waters 2695 HPLC connected to a Micromass Quattro Ultima mass spectrometer, operating with an electrospray ionisation source (ESI positive mode).

An HPLC-MS method utilizing a Phenomenex LUNA C18(2) 5µ reversed phase HPLC column (150 x 3.00 mm, flow rate 0.5 ml/min) was used. A gradient of 80 % H₂O + 0.1 % formic acid (A) and 20 % acetonitrile (B) was applied. Percentage of B was linearly increased from 20 % to 90 % within 10 min and then kept isocratic at 90 % B for 2 min.

MS data were acquired in the MRM mode (Parent ion: [M+H]⁺, daughter ion: 227.0). Up to 6 MRM traces of different masses were detected in parallel channels; for larger libraries repetitive injections of the same sample were necessary. Important MS source tuning parameters: Capillary 3 kV, cone 70 V, source temperature 140°C, desolvation temperature 400°C, ion energy 0.5, collision energy 7, multiplier 650 V.

Typical HPLC/MS method for dynamic hydrazone libraries without target and for binary dynamic libraries with target:
HPLC-MS analyses were performed with an Agilent 1100 HPLC connected to a Bruker Esquire 3000Plus ion trap mass spectrometer, operating with an electrospray ionisation source (ESI positive mode).

Compounds were eluted with 0.1 % formic acid in H₂O (A) : acetonitrile (B) from a Phenomenex LUNA C18(2) 5µ reversed phase HPLC column (250 x 3.00 mm, flow rate 0.5 ml/min). Gradients with increasing percentage of B (starting from 20 % and ending at max. 95 %) were applied. In experiment 1 water made weakly alkaline (pH 9.5) by a small amount of trimethylamine was used as eluent A instead of formic acid solution.

MS data were acquired in the full scan mode and the raw data scanned for protonated molecular ions [M+H]⁺ of scaffolds and potential products by generation of extracted ion chromatograms (EICs). Important MS source tuning parameters: Skimmer 35.9 V, capillary exit 160.66 V, Trap Drive 61.4, HV capillary 4500 V, dry temperature 200 °C, dry gas 8.0 1/min, nebulizer 220,6 kPa (32psi); ion trap parameters: scan range 100-1500 m/z, averages 8 spectra, max. accumulation time 50 ms, ion charge control on, ICC target 20000.

### Dynamic Combinatorial Libraries in the Absence of a Target (Comparative) Aldehyde Scaffold with Hydrazide Building Blocks

### Experiment 1

This is an experiment in which the distribution of excess hydrazide molecules **Hn** in their reaction with a trivalent scaffold (scheme 1) through hydrazone exchange in the absence of a target is kinetically determined.

In experiment A, a trialdehyde scaffold **SI** (120 µM) is reacted at pH 6.6 (10 mM ammonium acetate buffer) with a 1:1 mixture of 2 hydrazides **H11, H107** (R = OH, NO₂; 500 µM each) until equilibrium is reached. The so-formed library contains 4 different trisubstituted products (beside minor amounts of di- and monosubstituted products). At time t = 0 the library is spiked with a third hydrazide **H34** (R = H) at a concentration equal to each of the previous ones. The composition of the library is then monitored by HPLC/MS as a function of time. The incorporation of the R = H substituent into the trihydrazones can experimentally be confirmed by the appearance of 6 novel products most of which are already detectable after less than an hour. The library now consists of a total population of 4 + 6 = 10 different components (table 1). Under the assumption that reactions at all 3 functional groups of the scaffold are taking place independently of each other, the abundance of each individual product within this population is determined by its relative thermodynamic stability and the statistical probability factor of its particular substituent combination arising from the threefold symmetry of the scaffold. Experimentally measured peak area distributions are determined by a superposition of the factors mentioned above with their ionisation behaviour in the ESI-MS ion source.

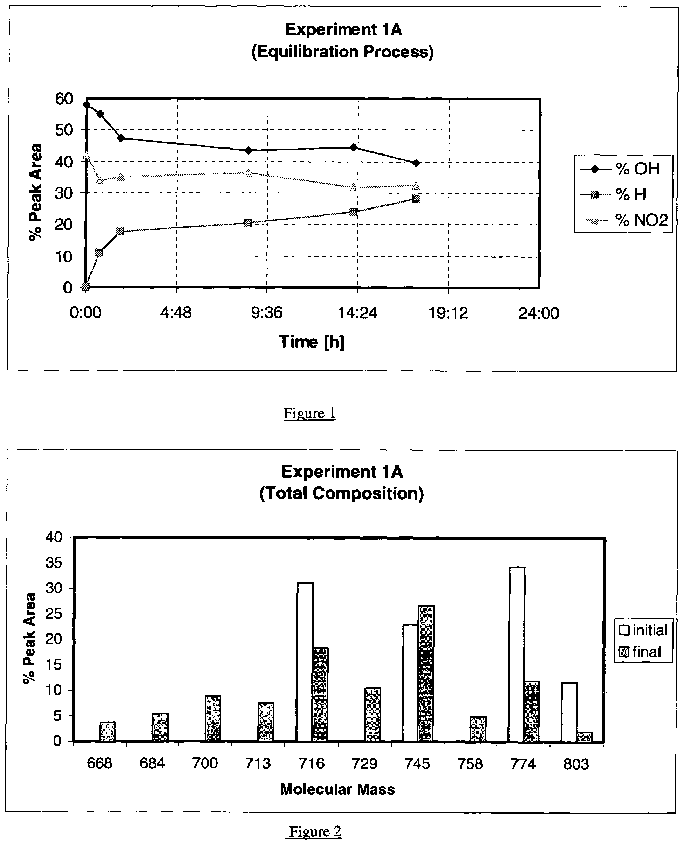

As can be seen in the diagram shown in figure 1, the total content of the hydrogen substituent (i.e., unsubstituted phenyl) increases with time at the cost of both initial hydroxy and nitro substituents which are gradually being displaced. The relative peak areas for all substituents level off after ca. 24 h which indicates that the new equilibrium state is reached. No significant changes occur thereafter (final substituent content after 6 days: 51 % OH, 21 % H, 28 % NO₂). Both initial and final product distributions are depicted together in a bar diagram (figure 2) for comparison.

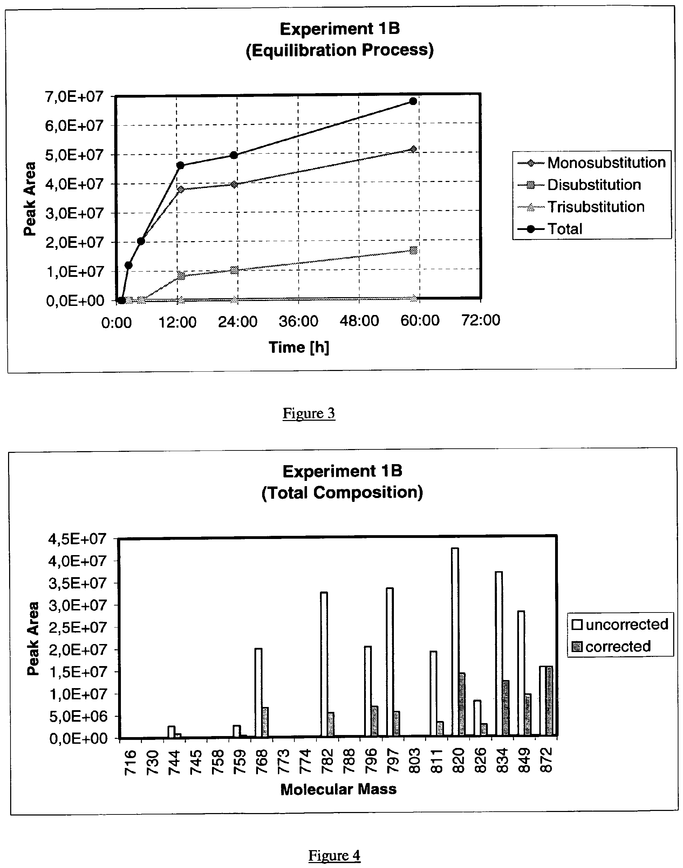

Experiment B is carried out in a similar way using the same scaffold **SI** but starting from an initial diversity of 20 components (4 hydrazides **H11, H106, H43, H107** (R = OH, OCH₃, CF₃, NO₂) at equal concentrations of 300 µM) and proceeding towards a final diversity of 20 + 15 = 35 components via spiking with the same additional hydrazide **H34** (300 µM) as before. The total hydrazide concentration after spiking is the same (1.5 mM) as in experiment A. Although not all 35 products (especially not those with low probability factors of formation) are detectable in the mixture anymore (figure 4; peak areas are given both as absolute numbers and "corrected", i.e. divided by the probability factor for the respective mass), the total amount of detected R = H products is again increasing with time with monosubstitution appearing faster than higher substitution degrees. For clarity reasons, peak areas of all newly formed individual products shown in figure 3 are partitioned into mono-, di- and trisubstitution by **H34** but summed up within each of these categories.

The principal difference between A and B is that the equilibration in B proceeds more slowly since each individual hydrazide building block is now available for exchange in a lower concentration only. Nevertheless, even such relatively large dynamic libraries can be equilibrated in almost neutral medium within approximately 3-4 days under the mild experimental conditions described in this application, as shown in the diagram by the concentration plateau reached. Since the addition of components to a dynamic library can be regarded as an equilibrium perturbation similar to the selective removal of such components (e.g., through target binding), it is expected that a target-induced shift will be complete within a similar timeframe in dependence of the size of the library.

**Table 1**

| Mass | Probability Factor | R=H | R = OH | R = NO₂ |
|---|---|---|---|---|
| | | | | |
| 668 | 1 | 3 | - | - |
| 684 | 3 | 2 | 1 | - |
| 700 | 3 | 1 | 2 | - |
| 713 | 3 | 2 | - | 1 |
| 716 | 1 | - | 3 | - |
| 729 | 6 | 1 | 1 | 1 |
| 745 | 3 | 1 | 2 | 1 |
| 758 | 3 | - | - | 2 |
| 774 | 3 | - | 1 | 2 |
| 803 | 1 | - | - | 3 |

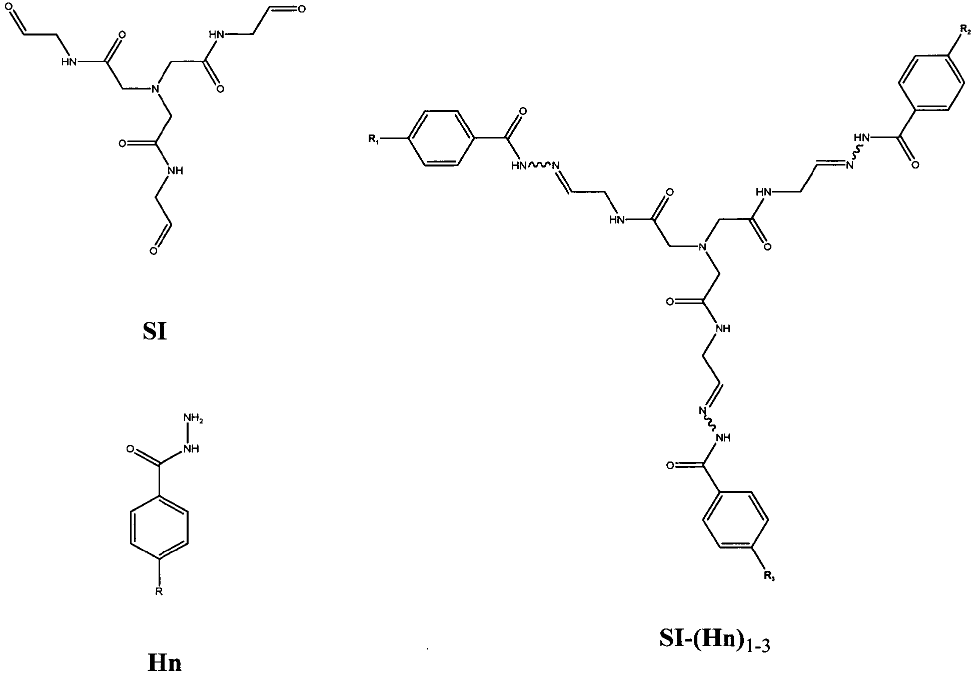

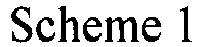

### Hydrazide Scaffold with Aldehyde Building Blocks

### Experiment 2

This is a kinetic experiment demonstrating the viability of the method described in this application also for the inverse reaction scheme, i.e. the equilibration of a hydrazine scaffold in the presence of excess aldehydes.

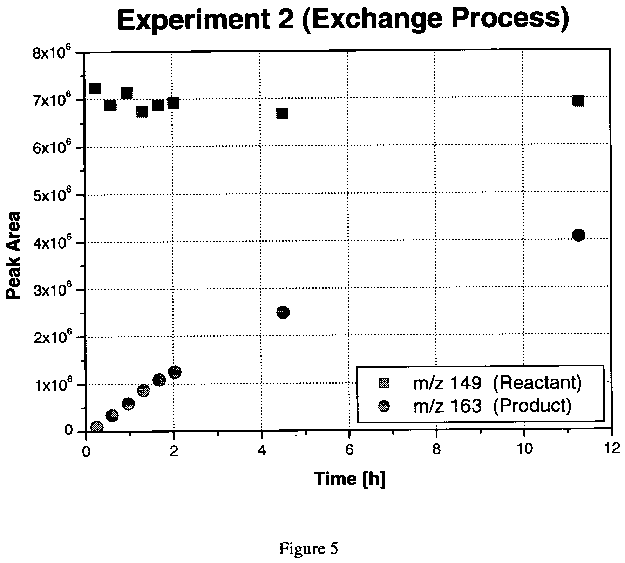

As a model study the reaction in scheme 2 is used in which N-aminopyridinium iodide (100 µM) was first treated at pH 5.5 with a 10fold excess of butyraldehyde (1 mM) for ca. 43 hours upon which it was converted into the hydrazone with m/z = 149, then an equal excess of a highly concentrated valeraldehyde solution was added. Since the reaction volume has not changed significantly the total aldehyde concentration is now ca. 20 times as high as that of the heteroaromatic hydrazine scaffold. Samples are taken from the equilibrating mixture at regular time intervals and analysed by HPLC/MS in order to monitor the formation of the exchange product with m/z = 163 (Figure 5).

Since MS signal calibration curves are usually nonlinear over a large concentration range, the decrease of the reactant does not fully match the product increase although other products are not formed in the reaction. Compared to the combination of an aldehyde scaffold and a library of hydrazide building blocks as shown in all previous experiments, it is obvious that equilibration needs longer time to proceed. Nevertheless, equilibration of such a binary library at pH 5.6 is complete by achieving almost equal peak areas of both hydrazones within 21 hours at the latest (not shown in the graph) which is acceptable for screening in the presence of a biological target.

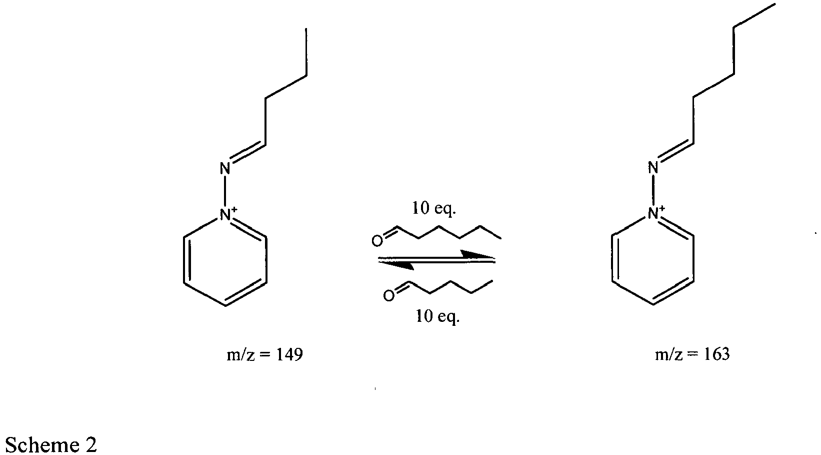

### Dynamic Combinatorial Libraries in the Presence of a Target

### Aldehyde Scaffold with Hydrazide Building Blocks

### Experiment 3

The following experiment demonstrates the usefulness of fully dynamic hydrazone libraries (experiment A) in comparison with hydrazone libraries that are essentially static in the screening step (e.g., pre-equilibrated libraries in which the hydrazone exchange has been stopped by shifting from pH 6.5 to pH 9.2 via addition of a very small amount of 1 M aqueous NH₃ solution prior to addition of the biological target; experiment B). Otherwise in both experiments the same 12-component hydrazone library **SII-Hn** (scheme 3) is used. Each hydrazide was present in a 250 µM concentration, the scaffold and target concentrations were 15 µM each.

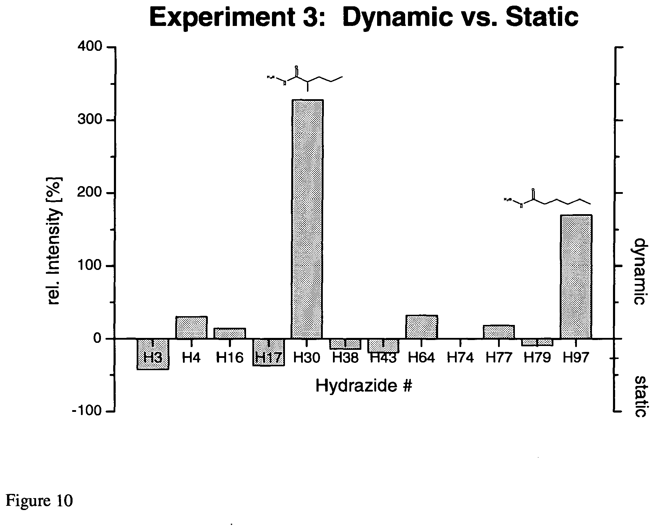

In both experiments A (figure 6) and B (figure 8) the compositions of the mixtures after ca. 1 and ca. 3 days show certain differences when the runs in the presence of the target are compared to the three control runs. In experiment B the differences are most pronounced with **SII-H77** and **SII-H79.** The two truly most active components, **SII-H30** (IC₅₀ = 200-500 nM) and **SII-H97** however, are unambiguously identified only in the fully dynamic experiment A which takes advantage of the enrichment in only the strongest binders. The increase of their peak areas in A is confidently above the level of reproducibility errors, i.e.the signal-to-noise ratio of the library analysis has been improved. This can be easily visualized if the peak area differences between both experiments are calculated and plotted for each hydrazone (figure 10).

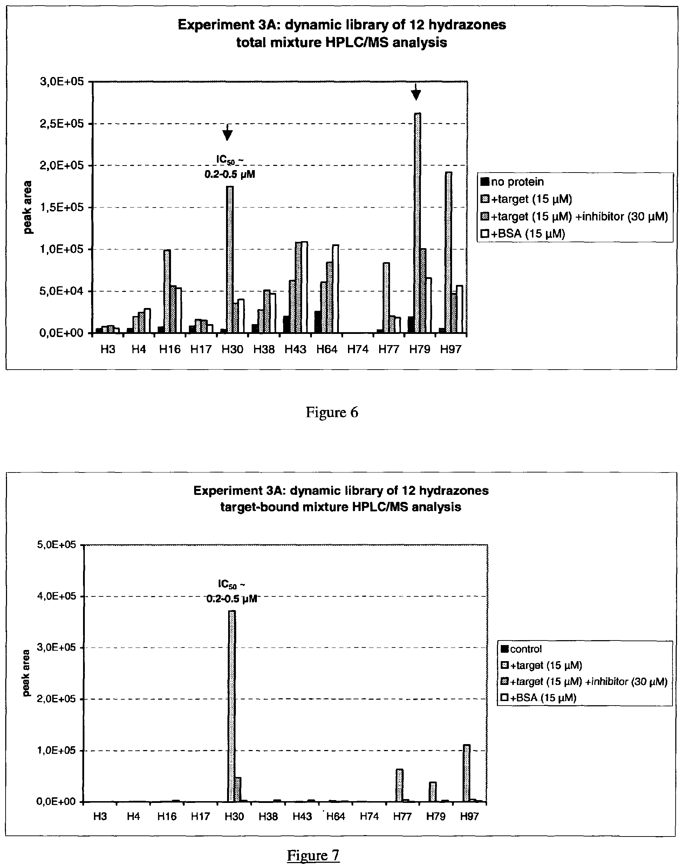

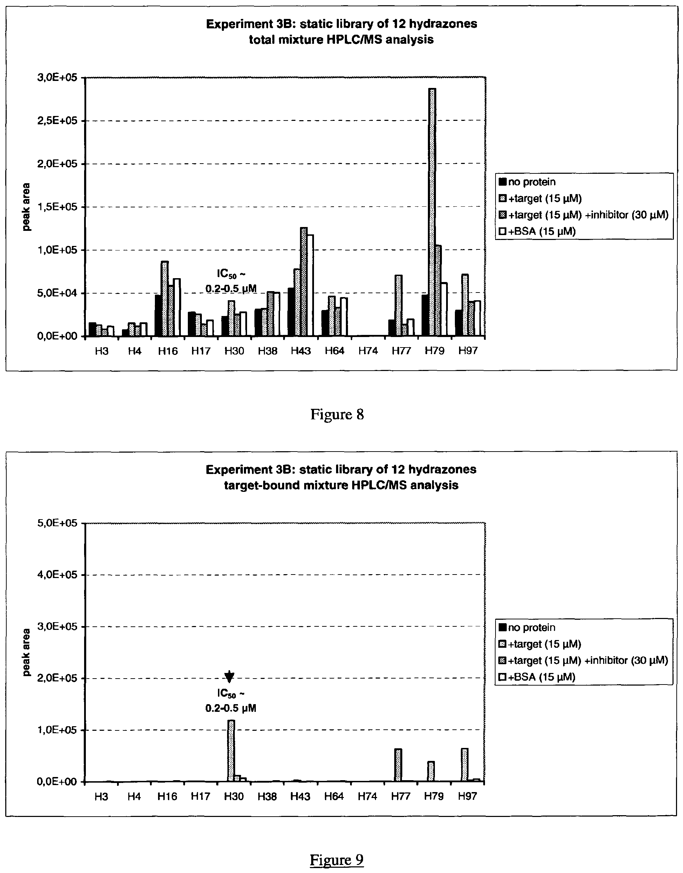

**SII-H30** is in both experiments the dominant component within the target-bound fraction (A: figure 7; B: figure 9) as determined by HPLC/MS after removal of the unbound fraction but it is hardly identifiable as a hit by simple HPLC/MS analysis of the static homogeneous mixture B without further deconvolution.

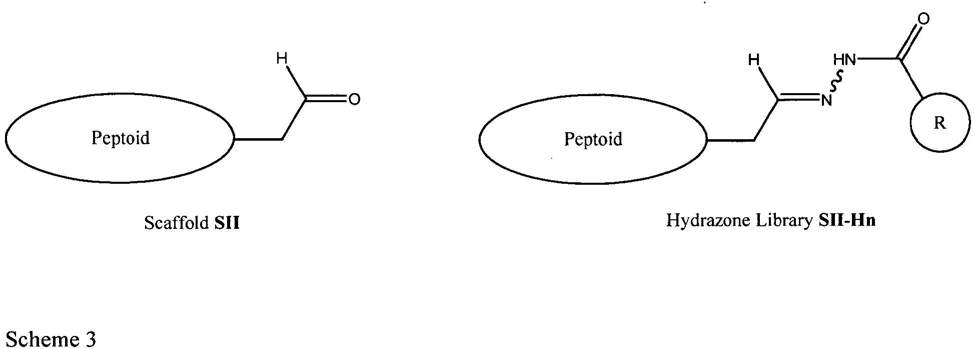

## Claims

1. A method of forming a dynamic library of compounds which are potentially capable of binding to a target, which method comprises
i) selecting one first set of molecules which molecules each carry at least one first functional group which is capable of interacting with at least one other functional group under the formation of a reversible bond;
ii) selecting at least one further set of molecules which molecules carry at least one second functional group which is capable of interacting with the first functional group on the first set of molecules;
iii) reacting the two sets of molecules with each other and in the presence of the target, under conditions where a formation of reversible bonds between the functional groups on the molecules forming each of the sets occurs;
wherein
a library containing products, that can be isolated and do not decompose when the set of molecules present in excess is removed, is generated,
the formation of the library is carried out in an aqueous medium at a pH of from 5 to 9 and is fully reversible throughout the entire formation of the library,
the formation of reversible bonds is the formation of hydrazones R¹C(R²)=N-N(R³)-D (I) by the reaction between a carbonyl function R¹C(O)R² (II) and a hydrazine function according to the general formula H₂N-N(R³)-D (III) wherein R¹ and R² are independently of each other hydrogen or an organic radical, R³ is hydrogen, an organic radical or an electron withdrawing group, and D is selected from the group -C(O)-, -C(O)-O-, -C(O)-S-, -C(O)-N<, -C(O)-C(O)-, - C(O)-(O)-O-, -C(O)-C(O)-N<, -C(S)-, -C(S)-S-, -C(S)-N<, -C(=N)-N<, -C(=N-CN)-N<, -C(=N-NO₂)-N<, -S(O)-, -S(O)₂-, -S(O)₂-O-, -S(O)₂-N<, -P(O)(-O-)(-O-), -P(O)(-O-)-, -P(O)-, or D forms together with the group R³ and the nitrogen atom to which it is attached a heterocycle,
and the respective amounts of each set of molecules employed are such that one functional group of that at least one second functional groups is present in an at least 5-fold excess with respect to the other functional group during the formation of the library, which means that an excess of hydrazine groups is used relative to carbonyl groups.

2. The method according to claim 1, wherein the excess is at least 50-fold.

3. The method according to claim 1 or 2, wherein the target is a biological macromolecule from the group of proteins including lipoproteins, glycoproteins and analogues of proteins.

4. The method according to any of claims 1 to 3, wherein the concentration of the set of molecules used in excess is in the range from 500 µM to 10 mM.

5. The method according to any of claims 1 to 4, wherein the exchange reaction between an existing chemical bond and a functional group present in the library occurs by a substitution mechanism.

6. The method according to claim 5, wherein the functional group which is used in excess is the functional group which attacks the chemical bond formed in the substitution reaction, under the formation of a new chemical bond.

7. The method according to any of claims 1 to 6, wherein the formation of the library is carried out at a pH of from 5.5 to 8.

8. The method according to any of claims 1 to 7, wherein the target is present throughout the entire process of the formation of the library.

9. The method according to any of claims 1 to 8, wherein the hydrazone of formula I has the structure of formula VIII and the groups R and R' can be any organic group.

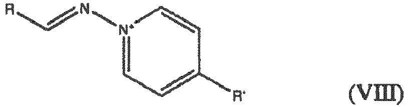

10. The method according to any of the claims 1 to 9, wherein a scaffold is used.

11. The method according to claim 10, wherein the target: scaffold ratio is kept at a molar ratio of between 0.5 : 1 and 3 : 1.

12. The method according to claim 10 or 11, wherein the scaffold carries at least one aldehyde functional group, the other set of molecules carries a hydrazine functional group and the hydrazine functional group is used in excess.

13. The method according to any of claims 10 to 12, wherein the scaffold carries two to six functional groups which are selected from the group consisting of carbonyl groups or hydrazine groups.

14. The method according to any of the claims 1 to 13, wherein the libraries are generated by reversible interconversion and equilibration in absence of the target, which is added in a second step when the library is still kept under equilibrium conditions and which is fully active.

15. A method of synthesizing and identifying a pharmaceutically active compound, which method comprises forming a library according to the method of any of claims 1 to 14, analysing the library by methods known as such and identifying the compound or compounds which preferably bind to the target.

16. The method according to claim 15, wherein the compound(s) binding preferably to the target is/are identified by comparing the amount of one or more compounds formed in the absence of the target with the respective amount formed in the presence of the target.

17. A method for the manufacture of a pharmaceutical preparation, comprising synthesizing and identifying a pharmaceutically active compound according to claim 15 or 16, optionally synthesizing and isolating the compound in amounts necessary for a medicament, and formulating the active compound and a pharmaceutically acceptable carrier into a pharmaceutical preparation.

## Patentansprüche

1. Verfahren zur Bildung einer dynamischen Bibliothek von Verbindungen, die potentiell in der Lage sind, an eine Zielstruktur zu binden, wobei besagtes Verfahren wie folgt umfaßt:
i) Auswahl einer ersten Zusammenstellung von Molekülen, welche jeweils mindestens eine erste funktionelle Gruppe tragen, die zur Wechselwirkung mit mindestens einer anderen funktionellen Gruppe unter Bildung einer reversiblen Bindung fähig ist;
ii) Auswahl mindestens einer weiteren Zusammenstellung von Molekülen, welche mindestens eine zweite funktionelle Gruppe tragen, die zur Wechselwirkung mit der ersten funktionellen Gruppe an der ersten Zusammenstellung von Molekülen fähig ist;
iii) Umsetzung der zwei Zusammenstellungen von Molekülen miteinander und in Gegenwart der Zielstruktur unter Bedingungen, unter denen eine Bildung von reversiblen Bindungen zwischen den funktionellen Gruppen an den Molekülen, die jede der Zusammenstellungen bilden, auftritt,
wobei
eine Bibliothek erzeugt wird, die Produkte enthält, welche isoliert werden können und sich nicht zersetzen, wenn die Zusammenstellung der Moleküle, die im Überschuß vorhanden sind, entfernt wird,
die Bildung der Bibliothek in einem wässrigen Medium bei einem pH von 5 bis 9 durchgeführt wird und während der gesamten Bildung der Bibliothek vollständig reversibel ist,
die Bildung reversibler Bindungen die Bildung von Hydrazonen R¹C(R²)=N-N(R³)-D (I) durch die Reaktion zwischen einer Carbonylfunktion R¹C(O)R² (II) und einer Hydrazinfunktion gemäß der allgemeinen Formel H₂N-N(R³)-D (III) ist, worin R¹ und R² unabhängig voneinander Wasserstoff oder einen organischen Rest bedeuten, R³ Wasserstoff, einen organischen Rest oder eine elektronenziehende Gruppe bedeutet und D aus der Gruppe -C(O)-, -C(O)-O-, -C(O)-S-, -C(O)-N<, -C(O)-C(O)-, -C(O)-(O)-O-, -C(O)-C(O)-N<, -C(S)-, -C(S)-S-, -C(S)-N<, -C(=N)-N<, -C(=N-CN)-N<, -C(=N-NO₂)-N<, -S(O)-, -S(O)₂-, -S(O)₂-O-, -S(O)₂-N<, -P(O)(-O-)(-O-), -P(O)(-O-)-, -P(O)- ausgewählt wird oder D zusammen mit der Gruppe R³ und dem Stickstoffatom, mit dem es verbunden ist, einen Heterozyklus bildet,
und die jeweiligen Mengen jeder verwendeten Zusammenstellung von Molekülen derart gestaltet sind, daß während der Bildung der Bibliothek eine funktionelle Gruppe jener mindestens einen zweiten funktionellen Gruppe in einem mindestens 5fachen Überschuß in Bezug auf die andere funktionelle Gruppe vorhanden ist, was bedeutet, daß ein Überschuß an Hydrazingruppen gegenüber Carbonylgruppen verwendet wird.

2. Verfahren nach Anspruch 1, wobei der Überschuß mindestens 50fach beträgt.

3. Verfahren nach Anspruch 1 oder 2, wobei die Zielstruktur ein biologisches Makromolekül aus der Gruppe der Proteine einschließlich der Lipoproteine, Glykoproteine und Proteinanalogen ist.

4. Verfahren nach einem der Ansprüche 1 bis 3, wobei die Konzentration der im Überschuß eingesetzten Zusammenstellung von Molekülen im Bereich von 500 µM bis 10 mM liegt.

5. Verfahren nach einem der Ansprüche 1 bis 4, wobei die Austauschreaktion zwischen einer bestehenden chemischen Bindung und einer in der Bibliothek vorhandenen funktionellen Gruppe über einen Substitutionsmechanismus verläuft.

6. Verfahren nach Anspruch 5, wobei die funktionelle Gruppe, die im Überschuß eingesetzt wird, diejenige funktionelle Gruppe ist, welche in der Substitutionsreaktion die entstandene chemische Bindung unter Ausbildung einer neuen chemischen Bindung angreift.

7. Verfahren nach einem der Ansprüche 1 bis 6, wobei die Bildung der Bibliothek bei einem pH von 5,5 bis 8 erfolgt.

8. Verfahren nach einem der Ansprüche 1 bis 7, wobei die Zielstruktur über den gesamten Verlauf der Bildung der Bibliothek hinweg zugegen ist.

9. Verfahren nach einem der Ansprüche 1 bis 8, wobei das Hydrazon der Formel I eine Struktur der Formel VIII aufweist und die Gruppen R und R' beliebige organische Gruppen darstellen können.

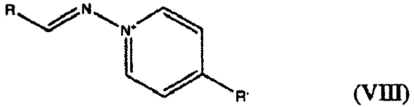

10. Verfahren nach einem der Ansprüche 1 bis 9, wobei eine Gerüststruktur verwendet wird.

11. Verfahren nach Anspruch 10, wobei das Verhältnis Zielstruktur :
Gerüststruktur in einem molaren Verhältnis zwischen 0,5 : 1 und 3 : 1 gehalten wird.

12. Verfahren nach Anspruch 10 oder 11, wobei die Gerüststruktur mindestens eine funktionelle Aldehydgruppe trägt, die andere Zusammenstellung von Molekülen eine funktionelle Hydrazingruppe trägt und die funktionelle Hydrazingruppe im Überschuß eingesetzt wird.

13. Verfahren nach einem der Ansprüche 10 bis 12, wobei die Gerüststruktur zwei bis sechs funktionelle Gruppen trägt, welche aus der Gruppe bestehend aus Carbonylgruppen oder Hydrazingruppen ausgewählt werden.

14. Verfahren nach einem der Ansprüche 1 bis 13, wobei die Bibliotheken durch reversible gegenseitige Umwandlung und Äquilibrierung in Abwesenheit der Zielstruktur erzeugt werden, welche in einem zweiten Schritt zugesetzt wird, während die Bibliothek immer noch unter Gleichgewichtsbedingungen gehalten wird und vollständig aktiv ist.

15. Verfahren zur Darstellung und Bestimmung einer pharmazeutisch wirksamen Verbindung, wobei besagtes Verfahren die Bildung einer Bibliothek gemäß dem Verfahren nach einem der Ansprüche 1 bis 14, die Analyse der Bibliothek auf an sich bekannte Art und Weise und die Ermittlung derjenigen Verbindung oder Verbindungen, welche bevorzugt an die Zielstruktur binden, umfaßt.

16. Verfahren nach Anspruch 15, wobei die bevorzugt an die Zielstruktur bindende(n) Verbindung(en) durch Vergleich der Menge einer oder mehrerer in Abwesenheit der Zielstruktur entstandener Verbindungen mit der entsprechenden, in Gegenwart der Zielstruktur entstandenen Menge ermittelt wird/werden.

17. Verfahren zur Herstellung einer pharmazeutischen Zubereitung umfassend die Darstellung und Bestimmung einer pharmazeutisch wirksamen Verbindung gemäß Anspruch 15 oder 16, gegebenenfalls die Darstellung und Abtrennung der Verbindung in für ein Arzneimittel erforderlichen Mengen, und die Formulierung der wirksamen Verbindung mit einem pharmazeutisch verträglichen Trägerstoff zu einer pharmazeutischen Zubereitung.

## Revendications

1. Un procédé de création d'une bibliothèque dynamique de composés potentiellement aptes à se lier à une cible, procédé qui comprend des actions suivantes
i) sélectionner un premier ensemble de molécules dans lequel chaque molécule porte au moins un premier groupement fonctionnel qui est apte à interagir avec au moins un autre groupement fonctionnel grâce à la formation d'une liaison réversible;
ii) sélectionner au moins un autre ensemble de molécules dans lequel les molécules portent au moins un second groupement fonctionnel qui est apte à interagir avec le premier groupement fonctionnel sur le premier ensemble de molécules;
iii) faire réagir les deux ensembles de molécules entre eux et en présence de la cible, dans des conditions où une formation de liaisons réversibles se produit entre les groupements fonctionnels sur les molécules formant chacun des ensembles,
dans lequel
est générée une bibliothèque contenant des produits pouvant être isolés et ne se décomposant pas lorsque l'ensemble des molécules présentes en excès est éliminé,
la création de la bibliothèque est réalisée dans un milieu aqueux avec un pH de 5 à 9 et est entièrement réversible tout au long de la création de la bibliothèque,
la formation de liaisons réversibles est la formation d'hydrazones R¹C(R²)=N-N(R³)-D (I) due à la réaction entre une fonction carbonyle R¹C(O)R² (II) et une fonction hydrazine selon la formule générale H₂N-N(R³)-D (III) dans laquelle R¹ et R² sont indépendamment l'un de l'autre hydrogène ou un radical organique, R³ est hydrogène, un radical organique ou un groupement électroattracteur et D est sélectionné à partir du groupe -C(O)-, -C(O)-O-, -C(O)-S-, -C(O)-N<, -C(O)-C(O)-, -C(O)-(O)-O-, -C(O)-C(O)-N<, -C(S)-, -C(S)-S-, -C(S)-N<, -C(=N)-N<, -C(=N-CN)-N<, -C(=N-NO₂)-N<, -S(O)-, -S(O)₂-, -S(O)₂-O-, -S(O)₂-N<, -P(O)(-O-)(-O-), -P(O)(-O-)-, -P(O)-, ou bien D forme avec le groupe R³ et l'atome d'azote auquel il est lié un hétérocycle,
et les quantités respectives de chaque ensemble de molécules utilisé sont telles qu'un groupement fonctionnel de ce au moins un second groupement fonctionnel est présent en excès au minimum de 5 fois par rapport à l'autre groupement fonctionnel au cours de la création de la bibliothèque, cela signifie qu'un excès de groupements hydrazine est utilisé par rapport aux groupements carbonyle.

2. Le procédé selon la revendication 1, dans lequel l'excès est au minimum de 50 fois.

3. Le procédé selon les revendications 1 ou 2, dans lequel la cible est une macromolécule biologique provenant du groupe de protéines comprenant des lipoprotéines, des glycoprotéines et des analogues de protéines.

4. Le procédé selon l'une quelconque des revendications 1 à 3, dans lequel la concentration de l'ensemble des molécules utilisées en excès est comprise entre 500 µM et 10 mM.

5. Le procédé selon l'une quelconque des revendications 1 à 4, dans lequel la réaction d'échange entre une liaison chimique existante et un groupement fonctionnel présent dans la bibliothèque se produit sous l'effet d'un mécanisme de substitution.

6. Le procédé selon la revendication 5, dans lequel le groupement fonctionnel qui est utilisé en excès est le groupement fonctionnel qui attaque la liaison chimique formée grâce à la formation d'une nouvelle liaison chimique lors de la réaction de substitution.

7. Le procédé selon l'une quelconque des revendications 1 à 6, dans lequel la création de la bibliothèque est effectuée à un pH de 5,5 à 8.

8. Le procédé selon l'une quelconque des revendications 1 à 7, dans lequel la cible est présente tout au long du processus de création de la bibliothèque.

9. Le procédé selon l'une quelconque des revendications 1 à 8, dans lequel l'hydrazone de formule I a la structure de la formule VIII et les groupes R et R' peuvent être n'importe quel groupement organique.

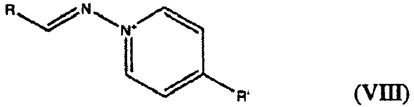

10. Le procédé selon l'une quelconque des revendications 1 à 9, dans lequel un échafaudage est utilisé.

11. Le procédé selon la revendication 10, dans lequel le rapport cible :
échafaudage est maintenu à un rapport molaire compris entre 0,5 : 1 et 3 : 1.

12. Le procédé selon les revendications 10 ou 11, dans lequel l'échafaudage porte au moins un groupement fonctionnel aldéhyde, l'autre ensemble de molécules porte un groupement fonctionnel hydrazine et le groupement fonctionnel hydrazine est utilisé en excès.

13. Le procédé selon l'une quelconque des revendications 10 à 12, dans lequel l'échafaudage porte de deux à six groupements fonctionnels choisis dans le groupe constitué par les groupements carbonyle ou les groupements hydrazine.

14. Le procédé selon l'une quelconque des revendications 1 à 13, dans lequel les bibliothèques sont générées par inter-conversion réversible et équilibration en l'absence de la cible, qui est ajoutée lors d'une seconde étape lorsque la bibliothèque continue d'être maintenue dans des conditions d'équilibre et est entièrement active.

15. Un procédé pour synthétiser et identifier un composé pharmaceutiquement actif, ce procédé comprend la création d'une bibliothèque selon le procédé de l'une quelconque des revendications 1 à 14, l'analyse de la bibliothèque par des méthodes connues pour le faire et l'identification du ou des composés qui se lient de préférence à la cible.

16. Le procédé selon la revendication 15, dans lequel le ou les composés se liant de préférence à la cible sont identifiés en comparant la quantité d'un ou plusieurs composés formés en l'absence de la cible avec la quantité correspondante formée en présence de la cible.

17. Un procédé pour la fabrication d'une préparation pharmaceutique comprenant la synthèse et l'identification d'un composé pharmaceutiquement actif selon les revendications 15 ou 16, facultativement en synthétisant et en isolant le composé en des quantités nécessaires pour un médicament, et la formulation du composé actif et d'un support pharmaceutiquement acceptable dans une préparation pharmaceutique.
